# EUROPEAN PATENT SPECIFICATION

(11) **EP 2 186 790 B1**
(45) Date of publication and mention of the grant of the patent: **13.04.2016**
(21) Application number: 08828264.5
(22) Date of filing: 20.08.2008
(51) Int. Cl.: C07C 45/52, C07C 45/82, C07C 47/22, C07C 51/235, C07C 57/055, C07C 51/25

(54) **ACROLEIN PRODUCTION METHOD AND ACRYLIC ACID PRODUCTION METHOD**
VERFAHREN ZUR HERSTELLUNG VON ACROLEIN UND VERFAHREN ZUR HERSTELLUNG VON ACRYLSÄURE
PROCÉDÉ DE PRODUCTION D'ACROLÉINE ET PROCÉDÉ DE PRODUCTION D'ACIDE ACRYLIQUE

(30) Priority: 29.08.2007 JP 2007223067; 29.08.2007 JP 2007223068; 29.08.2007 JP 2007223069
(43) Date of publication of application: 19.05.2010
(73) Proprietor: Showa Denko K.K., Tokyo 105-8518 (JP)
(72) Inventor: AOKI, Takanori, Tokyo 105-8518 (JP); ARAI, Norihide, Tokyo 105-8518 (JP)
(74) Representative: Strehl Schübel-Hopf & Partner
(86) International application number: PCT/JP2008/064828
(87) International publication number: WO 2009/028371

(56) References cited:
- EP-A1- 1 860 090
- WO-A1-2008/061860
- WO-A1-2008/066082
- WO-A2-2006/087084
- WO-A2-2006/092272
- JP-A- 2008 174 544

## Description

### TECHNICAL FIELD

The present invention relates to a method for producing acrolein or acrylic acid by using a glycerin mixture comprising glycerin as a main component.

### BACKGROUND ART

Generally speaking, acrolein and acrylic acid are produced by oxidation of propylene which is a fossil resource. However, production methods depending on fossil resources involve a concern that carbon dioxide increases in the atmosphere. In addition, exhaustion of fossil resources is also anticipated in future.

Therefore, studies are being made for the production of acrolein or acrylic acid by using glycerin that is produced as a by-product during the production of biodiesel fuels which serves as substitutes for fossil resources, or during the production of soaps, from vegetable or animal fats and oils. In addition, methods for producing acrolein through dehydration of by-product glycerin are also being studied.

Here, glycerin produced from vegetable fats and oils does not involve the concern of resource exhaustion since it is originated from plants. Furthermore, its carbon source is atmospheric carbon dioxide. Therefore, advantageously, substantially no contribution is given to the increase of carbon dioxide in the atmosphere. In addition, animal fats and oils are resources produced by livestock which intakes vegetable fats and oils or like feedstuffs. Therefore, their carbon source can also be considered as atmospheric carbon dioxide.

There is a known conventional method for producing acrolein from glycerin, in which acrolein is produced by dehydrating glycerin in a liquid or vapor phase with the presence of a solid catalyst (for example, see Patent Document 1). In addition, Patent Document 2 discloses a method for producing acrylic acid by vapor phase oxidation of acrolein produced by glycerin dehydration (for example, see Patent Document 2). In these reactions, usually, high purity glycerin is used.
Patent Document 1: Japanese Unexamined Patent Application, First Publication No. H 6-211724
Patent Document 2: Japanese Unexamined Patent Application, First Publication No. 2005-213225

WO 2008/061860 A1 is prior art pursuant to Ar.t 54(3) EPC and discloses a process for preparing alkyl esters of fatty acids and acrolein form triglycerides via intermediate formation of glycerine in the reaction mixture.

EP 1 860 090 A1 is prior art pursuant to Ar.t 54(3) EPC and discloses a process for treating a glycerol fraction from fatty acids transesterification that comprises transesterification byproducts to afford acrolein.

JP 2008 174544 A, WO 2006/092272 A2, and WO 2006/087084 A2 disclose a process for the preparation via dehydration of acrolein form glycerine.

WO 2008/066082 A1 is prior art pursuant to Ar.t 54(3) EPC and discloses a process for making acrolein from glycerine via dehydration in which raw glycerine mixture comprises as well fatty acids or esters.

### DISCLOSURE OF INVENTION

Glycerin produced during the production of biodiesel fuels, or during the production of soaps, from vegetable or animal fats and oils, contains impurities such as by-product fatty acids and fatty acid salts. For this reason, distillation is a must to obtain high purity glycerin. However, since the boiling point of glycerin is high, distillation of glycerin requires a large amount of energy. If a large amount of energy has to be consumed for the utilization of by-product glycerin, it is almost meaningless to use by-product glycerin.

From these situations, there is a demand for the utilization of by-product glycerin produced during the production of biodiesel fuels or during the production of soaps, with a little consumption of energy.

It is an object of the present invention to provide an acrolein production method capable of producing acrolein from a glycerin mixture (comprising glycerin; and at least one selected from the group consisting of a glyceride, a fatty acid ester, and either one or both of a fatty acid and a fatty acid salt, hereunder referred to as the "glycerin mixture"), which is produced for example during the production of biodiesel fuels, or during the production of soaps, with a little consumption of energy.

Moreover, it is also an object of the present invention to provide an acrylic acid production method capable of producing acrylic acid from the glycerin mixture with a little consumption of energy.

The inventors of the present invention have earnestly conducted studies, as a result of which they discovered that acrolein was able to be produced by dehydrating glycerin even with the coexistence of impurities such as a glyceride, a fatty acid ester, and either one or both of a fatty acid and a fatty acid salt, at an equivalent yield as compared to the case without the coexistence of such impurities. This has led to the development of the acrolein production method and the acrylic acid production method of the present invention.

That is, the present invention takes the following structures.

[1] An acrolein production method comprising: performing, either concurrently or sequentially,
   (1) a step of dehydrating a glycerin mixture comprising the following (i) and (ii):
      (i) 5 to 95 % by mass of glycerin; and
      (ii) at least one selected from the group consisting of: a glyceride; either one or both of a fatty acid and a fatty acid salt; and a fatty acid ester,
      to produce an acrolein mixture comprising the following (iii) and (iv):
      (iii) acrolein; and
      (iv) at least one selected from the group consisting of: a glyceride; a fatty acid ester; and either one or both of a fatty acid and a fatty acid salt; and
   (2) a step of collecting acrolein from the acrolein mixture;
      wherein the acrolein production method further comprises,
      prior to said step (1) of producing the acrolein mixture:
      (a) a step of transesterifying a fat/oil and an alcohol, to produce a fatty acid ester mixture comprising: a fatty acid ester; and at least any one of glycerin, a glyceride, and either one or both of a fatty acid and a fatty acid salt; and
      (b) a step of removing the fatty acid ester from the fatty acid ester mixture, to thereby produce the glycerin mixture, in which the mass ratio of fatty acid ester to glycerin is 0.001-1:1; and
      after said step (2) of collecting acrolein from the acrolein mixture:
      treating a part or a whole of the remaining residue with an acid and returning the treated residue to the step (a) of producing the fatty acid ester mixture;
      or
      wherein the acrolein production method further comprises,
      prior to said step (1) of producing the acrolein mixture:
      (c) a step of saponifying a fat/oil and an alkali, to produce a fatty acid alkaline salt mixture comprising a fatty acid alkaline salt; glycerin, and either one or both of a fatty acid and a fatty acid salt; and
      (d) a step of removing the fatty acid alkaline salt from the fatty acid alkaline salt mixture, to produce the glycerin mixture, in which the ratio of the fatty acid alkaline salt to glycerin is 0.001-1:1; and
      after said step (2) of collecting acrolein from the acrolein mixture:
      returning a part or a whole of the remaining residue to the step (c) of producing the fatty acid alkaline salt mixture.
[2] An acrolein production method according to [1] above, wherein the step (2) of collecting acrolein from the acrolein mixture is carried out by distillation of the acrolein mixture.
[3] An acrolein production method according to [1] or [2], wherein said fatty acid comprises one or more types of fatty acid(s) selected from the group consisting of C4 to C24 fatty acids; said fatty acid salt comprises salt(s) of one or more types of fatty acid(s) selected from the group consisting of C4 to C24 fatty acids, and one or more types of compound(s) selected from the group consisting of an alkali metal compound, an alkali earth metal compound, and an amine compound; and the fatty acid constituting said glyceride comprises one or more types of fatty acid(s) selected from the group consisting of C4 to C24 fatty acids.
[4] An acrylic acid production method, comprising a) the acrolein production method according to any one of [1] to [3] above and b) reacting the acrolein produced in step a) with molecular oxygen.

The acrolein production method of the present invention is capable of producing acrolein from a glycerin mixture with a little consumption of energy.

Furthermore, the acrylic acid production method of the present invention is capable of producing an acrylic acid from a glycerin mixture with a little consumption of energy.

### BEST MODE FOR CARRYING OUT THE INVENTION

### [Acrolein production method]

### <First exemplary embodiment>

Here is a description of a first exemplary embodiment of the acrolein production method of the present invention.

The acrolein production method of this exemplary embodiment comprises: a step of transesterifying a fat/oil and an alcohol, to thereby produce a fatty acid ester mixture (hereinunder, referred to as the first step); a step of removing the fatty acid ester from the fatty acid ester mixture, to thereby produce a glycerin mixture (hereinunder, referred to as the second step); a step of dehydrating the glycerin mixture, to thereby produce an acrolein mixture (hereinunder, referred to as the third step); a step of collecting acrolein from the acrolein mixture (hereinunder, referred to as the fourth step); and a step of appropriately treating a part or a whole of the residue remaining after the fourth step with an acid, and returning the treated residue back to the first step (hereinunder, referred to as the fifth step).

### (First step)

The fat/oil in the first step can be exemplified by a vegetable fat/oil, an animal fat/oil, and a waste fat/oil.

The vegetable fat/oil can be exemplified by flaxseed oil, safflower oil, sunflower oil, soybean oil, corn oil, peanut oil, cottonseed oil, sesame oil, rice oil, rapeseed oil, olive oil, palm oil, palm kernel oil, coconut oil, castor oil, rice bran oil, walnut oil, camellia oil, and peanut oil.

The animal fat/oil can be exemplified by beef tallow, lard, mutton tallow, neatsfoot oil, bird oil, chicken oil, fish oil, whale oil, and butter.

The waste fat/oil can be exemplified by animal and vegetable fats and oils used for cooking at domestic sites, restaurants, fast food shops, bento (packed meal) production factories, and school meal factories.

Here, the term "fat/oil" refers to an ester of a fatty acid and glycerin. Moreover, the fatty acid means a monocarboxylic acid of a long chain hydrocarbon. The long chain hydrocarbon may have a double bond. Preferred examples of the fatty acid suitable for the present invention include one or more types of fatty acid(s) selected from the group consisting of C4 to C24 fatty acids. Specific examples of C4 to C24 fatty acids can include butyric acid, caproic acid, caprylic acid, capric acid, lauric acid, myristic acid, palmitic acid, stearic acid, arachic acid, behenic acid, lignoceric acid, oleic acid, cetoleic acid, erucic acid, brassidic acid, sorbic acid, linoleic acid, linolenic acid, arachidonic acid, eicosenoic acid, and ricinoleic acid.

Preferred examples of the alcohol in the first step suitable for the present invention include one or more types of alcohol(s) selected from the group consisting of C 1 to C10 alcohols. Specific examples of C1 to C10 alcohols can include methylalcohol, ethylalcohol, n-propylalcohol, iso-propylalcohol, n-butylalcohol, iso-butylalcohol, sec-butylalcohol, tert-butylalcohol, n-pentylalcohol, n-hexylalcohol, n-heptylalcohol, n-octylalcohol, n-nonylalcohol, and n-decylalcohol.

Upon the transesterification reaction, it is preferable in terms of the productivity to use a transesterification catalyst. The transesterification catalyst includes acid catalysts and basic catalysts. The acid catalysts can be exemplified by inorganic acids such as sulfuric acid, hydrochloric acid, and phosphoric acid; strong acid ion-exchange resins, heteropoly acids such as silicotungstic acid and phosphotungstic acid, and zirconium sulphate. The basic catalysts can be exemplified by hydroxides, oxides, carbonates, or alkoxides of such alkali metals as sodium, potassium, rubidium, and cesium; hydroxides, oxides, carbonates, or alkoxides of such alkali earth metals as magnesium, calcium, strontium, and barium; strong basic anion-exchange resins, and amines.

Transesterification between a fat/oil and an alcohol yields a fatty acid ester mixture which comprises a fatty acid ester, glycerin, a glyceride, and either one or both of a fatty acid and a fatty acid salt. The fatty acid ester produced herein correspond to the fat/oil and the alcohol used, which can be used as a fuel for diesel engines, so-called a biodiesel fuel.

Moreover, the fatty acid ester is an ester of a fat/oil-constituting fatty acid and an alcohol. Preferred examples of the fatty acid ester suitable for the present invention include esters of one or more types of fatty acid(s) selected from the group consisting of C4 to C24 fatty acids, and one or more types of alcohol(s) selected from the group consisting of C1 to C10 alcohols, and preferably C1 to C4 alcohols.

Furthermore, the fatty acid ester preferably has its boiling point within a range of 200 to 400°C at 0.10 MPa. Examples of the fatty acid ester having such a boiling point can include methyl caproate, methyl caprylate, methyl caprate, methyl laurate, methyl myristate, methyl palmitate, methyl stearate, methyl oleate, methyl linolate, methyl linoleate, and methyl ricinoleate. These fatty acid esters are difficult to separate from glycerin by distillation since their boiling points are close to that of glycerin. However, the present invention can facilitate the separation thereof through conversion of glycerin into acrolein.

The glyceride may be any one of a monoglyceride, a diglyceride, and a triglyceride. The fatty acid(s) constituting the glyceride is/are the same as the fat/oil-constituting fatty acid(s) mentioned above.

### (Second step)

The method for removing the fatty acid ester from the fatty acid ester mixture in the second step can be exemplified by distillation, liquid-liquid separation, and column separation of the fatty acid ester mixture.

The glycerin mixture produced by removing the fatty acid ester from the fatty acid ester mixture contains glycerin and either one or both of fatty acid(s) and fatty acid salt(s). The glycerin mixture may also contain components other than glycerin and the fatty acid ester, such as water, a base, an acid, a glyceride, a fatty acid, a fatty acid salt, and an alcohol. In addition, the glycerin mixture may also be diluted with a solvent which does not inhibit reactions in the third and the following steps (for example, water).

The glycerin content in the glycerin mixture produced by removing the fatty acid ester from the fatty acid ester mixture is 5 to 95% by mass, more preferably 10 to 95% by mass, and particularly preferably 15 to 95% by mass. The glycerin content is not less than 5% by mass, in terms of securing an enough yield of acrolein. The present invention is of more use at a glycerin content of not higher than 95% by mass.

Even if the fatty acid ester has been separated and removed from the fatty acid ester mixture, the removal can not be perfectly completed. That is, the produced glycerin mixture still contains a small amount of the fatty acid ester.

The content of the fatty acid ester in the glycerin mixture is 0.001 to 1, and preferably 0.01 to 0.1, in the mass ratio assuming that the glycerin content (by mass) is 1. If the fatty acid ester content is not greater than 0.001 assuming that the glycerin content is 1, the present invention tends to be of less use. If the fatty acid ester content exceeds 1, the acrolein yield will be so small that the efficiency tends to decrease.

The fatty acid(s) contained in the glycerin mixture are the same as the fat/oil-constituting fatty acid(s). Here, the fatty acid contained in the glycerin mixture preferably has its boiling point within a range of 200 to 400°C at 0.10 MPa. Examples of the fatty acid having such a boiling point can include caproic acid, caprylic acid, capric acid, lauric acid, myristic acid, palmitic acid, stearic acid, oleic acid, linoleic acid, linolenic acid, and ricinoleic acid. These fatty acids are difficult to separate from glycerin by distillation since their boiling points are close to that of glycerin. However, the present invention can facilitate the separation thereof through conversion of glycerin into acrolein.

Moreover, the fatty acid salt contained in glycerin comprises salt(s) of one or more types of fatty acid(s) selected from said group consisting of C4 to C24 fatty acids, and one or more types of compound(s) selected from the group consisting of an alkali metal compound, an alkali earth metal compound, and an amine compound.

Here, the alkali metal compound can be exemplified by a hydroxide, an oxide, a carbonate, or an alkoxide of such alkali metals as sodium, potassium, rubidium, and cesium. The alkali earth metal compound can be exemplified by a hydroxide, an oxide, a carbonate, or an alkoxide of such alkali earth metals as magnesium, calcium, strontium, and barium.

In addition, the amine compound can be exemplified by ammonia, methylamine, dimethylamine, trimethylamine, ethylamine, diethylamine, triethylamine, propylamine, dipropylamine, tripropylamine, butylamine, dibutylamine, tributylamine, aniline, ethylenediamine, diethylenetriamine, pyrrole, pyridine, tetramethyl ammonium hydroxide, tetraethyl ammonium hydroxide, tetrapropyl ammonium hydroxide, tetrabutyl ammonium hydroxide, and urea.

### (Third step)

In the dehydration reaction of the glycerin mixture in the third step, specifically glycerin in the glycerin mixture is dehydrated to thereby produce acrolein.

In the dehydration reaction, it is preferable to use a dehydration catalyst for accelerating the reaction speed. Dehydration catalysts that can be used herein include acid catalysts and basic catalysts. The acid catalysts can be exemplified by natural or synthetic clay compounds of kaolinite, bentonite, montmorillonite, zeolite, or the like; oxides or oxide complexes of silica, alumina, titania, zirconia, silica-alumina, or the like; heteropoly acids, sulfates, acid sulfates, carbonates, acid carbonates, nitrates, acid nitrates, phosphate, acid phosphates, sulfuric acid, phosphoric acid, and acid ion-exchange resins.

In addition, supported catalysts in which a heteropoly acid, a sulfate, an acid sulfate, a carbonate, an acid carbonate, a. nitrate, an acid nitrate, a phosphate, an acid phosphate, sulfuric acid, or phosphoric acid is held by a carrier may also be used. The carrier can be exemplified by oxides or oxide complexes of silica, alumina, titania, zirconia, silica-alumina, or the like.

The form of the catalyst is not specifically limited, and examples thereof can include a powder form, a globular form, a columnar form, a saddle form, and a honeycomb form.

The method for preparing the catalyst can be exemplified by an impregnation method, a precipitation method, and an ion exchange method.

Moreover, the catalyst may also be previously baked in gas according to the purpose. Examples of the gas can include nitrogen, argon, helium, and air.

The dehydration reaction may be either a liquid phase reaction or a vapor phase reaction. The reaction may be performed by any one of batch operation, semibatch operation, and continuous operation.

The dehydration reaction can be carried out at a temperature of 0 to 600°C. Preferred temperature is 100 to 500°C, and more preferred temperature is 150 to 400°C, as it offers high reaction efficiency.

Since the dehydration reaction of glycerin is a reaction in which the number of moles increases, the glycerin yield increases as the pressure decreases. Specifically preferred pressure is 0.01 to 10.0 MPa, and more preferred pressure is 0.05 to 5 MPa.

However, in a case of the liquid phase reaction, the temperature and the pressure are selected so that glycerin and fatty acid(s) can be present in a liquid form. In a case of the vapor phase reaction, the temperature and the pressure are selected so that glycerin and fatty acid(s) can be present in a vapor form.

In a case of the liquid phase reaction, a solvent may be used. The solvent is preferably stable at the reaction temperature. Such a solvent can be exemplified by such saturated hydrocarbons as liquid paraffin, paraffin wax, dodecane, tridecane, tetradecane, and hexadecane, such aromatic hydrocarbons as dibenzyl; diphenyl ether, sulfolane, and silicone oil.

In a case of the vapor phase reaction, an inert gas may be used for dilution. Usable inert gas can be exemplified by nitrogen, carbon dioxide, noble gas (such as helium and argon), and water vapor.

The content of acrolein in the acrolein mixture produced by the third step is determined depending on the glycerin content in the glycerin mixture, the yield constant of the dehydration reaction, and the like. Specifically preferred is within 5 to 60% by mass.

### (Fourth step)

As to the method for collecting acrolein from the acrolein mixture, known separation/collection methods can be applied. However, in order to collect acrolein on the industrial basis, collection is preferably done by distillation of the acrolein mixture.

Specific examples of this collection method by distillation can include simple distillation, multistage distillation, steam distillation, and flash distillation. The distillation may be performed by any one of batch operation, semibatch operation, and continuous operation.

If the multistage distillation is applied, for example, low boiling point components having a boiling point lower than that of acrolein can be distilled off from acrolein from the top of the column, acrolein can be distilled off from the intermediate part, and glyceride(s) and either one or both of fatty acid(s) and fatty acid salt(s) can be distilled off from the bottom of the column.

As to the distillation column used in the multistage distillation, known distillation columns such as a tray type distillation column and a packed distillation column can be used.

Examples of the tray structure of the tray type distillation column can include a bubble cap tray, a sieve tray, a valve tray, a Superfrac tray, and a Maxfrac tray.

Examples of the packing in the packed distillation column can include regular packings and irregular packings. The regular packings can be exemplified by a metal plate, a metal mesh, and a grid. The irregular packings can be exemplified by a Raschig ring, a Lessing ring, a Berl saddle, an Intalox saddle, a Teralet packing, a Pall ring, a Flexi-ring, and a Cascade ring.

Regarding the condition for the distillation, the temperature at the bottom of the column can be set at 0 to 600°C. Particularly preferred temperature is 0 to 100°C, more preferably 5 to 80°C, and yet more preferably 10 to 60°C. If the temperature at the bottom of the column is higher than 100°C, acrolein may be polymerized. If the temperature at the bottom of the column is lower than 0°C, the energy amount required for cooling tends to be incremented. The pressure at the time of distillation is determined depending on the relation with the temperature.

Upon the collection of the acrolein mixture by distillation, it is preferable to add a polymerization inhibitor in advance so as to prevent the polymerization of acrolein. The polymerization inhibitor can be exemplified by phenothiazine, phenol, hydroquinone, methoquinone, catechol, cresol, and other phenolic compounds. The addition amount of the polymerization inhibitor, if added, is preferably 1 mass ppm to 1 mass % assuming that the acrolein amount is 100 mass %.

The fourth step may be performed either concurrently or after the third step.

A specific embodiment for performing the fourth step concurrently with the third step can be such that, for example, a glycerin mixture is supplied to a reactor comprising a distillation column where dehydration is carried out by liquid phase reaction to thereby produce acrolein, while at the same time the produced acrolein is collected from the top or the lateral side of the distillation column.

If the fourth step is performed concurrently with the third step, the reaction temperature of the third step can be set as the temperature at the bottom of the distillation column of the fourth step. For example, preferred temperature at the bottom of the column is within 100 to 500°C, and more preferred temperature is 150 to 400°C, in terms of maintaining high reaction efficiency. The pressure at the time of distillation is determined depending on the relation with the temperature.

The acrolein produced by the fourth step can be used as a raw material of acrylic acid, methionine, or 1,3-propanediol, for example.

### (Fifth step)

The residue remaining after the fourth step contains glyceride(s) and either one or both of fatty acid(s) and fatty acid salt(s). When a part of the residue is to be returned, a part of the mixture containing both the fatty acid and the fatty acid salt may be returned, or either the fatty acid alone or the fatty acid salt alone may also be returned.

If a part or a whole of the residue has been returned back to the first step, or if the fatty acid within the residue has been returned back, the fatty acid and the alcohol are esterified to produce a fatty acid ester.

The reason is that the transesterification catalyst used in the first step also functions as an esterification catalyst.

If the fatty acid salt within the residue is returned back to the first step, the fatty acid salt is used for the reaction with the fat/oil. In addition, if the fatty acid salt is returned to the first step, it is preferable to pretreat the fatty acid salt with an acid prior to the first step.

### <Second exemplary embodiment>

Here is a description of a second exemplary embodiment of the acrolein production method of the present invention.

The acrolein production method of this exemplary embodiment comprises: a step of saponifying a fat/oil and an alkali, to thereby produce a fatty acid alkaline salt mixture (hereinunder, referred to as the step (1')); a step of removing the fatty acid alkaline salt from the fatty acid alkaline salt mixture, to thereby produce a glycerin mixture (hereinunder, referred to as the step(2')); a step of dehydrating the glycerin mixture, to thereby produce an acrolein mixture (the third step); a step of collecting acrolein from the acrolein mixture (the fourth step); and a step of returning a part or a whole of the residue remaining after the fourth step back to the step (1') (hereinunder, referred to as the step(5')).

### (Step (1'))

The term "fat/oil" in the step (1') refers to the same meaning as the "fat/oil" described in the first exemplary embodiment.

The alkali for the reaction with the fat/oil in the first' step can be exemplified by a hydroxide, an oxide, a carbonate, or an alkoxide of such alkali metals as sodium, potassium, rubidium, and cesium; a hydroxide, an oxide, a carbonate, or an alkoxide of such alkali earth metals as magnesium, calcium, strontium, and barium; a strong basic anion-exchange resin, and an amine.

The fat/oil and the alkali can be reacted by any known method. The fatty acid alkaline salt mixture produced by this reaction contains fatty acid alkaline salt(s), glycerin, and either one or both of fatty acid(s) and fatty acid salt(s) corresponding to the alkali.

The fatty acid alkaline salt can be exemplified by fatty acid sodium, fatty acid potassium, fatty acid magnesium, and fatty acid calcium. These fatty acid alkaline salts can be used as soap.

### (Step (2'))

The method for removing the fatty acid alkaline salt from the fatty acid alkaline salt mixture in the second' step is the same as the method for removing the fatty acid ester from the fatty acid ester mixture in the first exemplary embodiment.

In the glycerin mixture produced by removing the fatty acid alkaline salt from the fatty acid alkaline salt mixture, preferred ranges of the glycerin content, the fatty acid content, and the fatty acid salt content are the same as those of the first exemplary embodiment.

Moreover, the glycerin mixture of this exemplary embodiment may also contain components other than glycerin, the fatty acid, and the fatty acid salt, such as water, a base, an acid, a fatty acid ester, an alcohol, and a glyceride. In addition, the glycerin mixture may also be diluted with a solvent which does not inhibit reactions in the third and the following steps (for example, water).

### (Third step and Fourth step)

The third step and the fourth step are the same as those of the first exemplary embodiment.

### (Step (5'))

If a part or a whole of the residue remaining after the fourth step is returned back to the step (1'), or if either one or both of the fatty acid and the glyceride is/are returned back to the step (1'), saponification reaction with alkali can be further generated.

In this embodiment, if the fatty acid salt is returned back to the step (1'), it is preferable to pretreat the fatty acid salt with an acid prior to the step (1').

In the acrolein production method of the first exemplary embodiment and the second exemplary embodiment mentioned above, since the glycerin mixture is subjected to the dehydration reaction without distillation, the glycerin mixture can be utilized with a little consumption of energy. Moreover, though a large consumption of energy is needed for separating glycerin having a high boiling point (boiling point at 0.1 MPa: 290°C) from glycerides and either one or both of fatty acids and fatty acid salts by distillation or like manner, only a small consumption of energy is needed for separating acrolein having a low boiling point (boiling point at 0.1 MPa: 53°C) from glycerides and either one of fatty acids and fatty acid salts, since acrolein can be distilled out.

Accordingly, this production method is capable of producing acrolein from the glycerin mixture with a little consumption of energy.

Furthermore, in these exemplary embodiments, since in the fifth step or the step (5') the residue remaining after the fourth step is returned back to the first step, the yield of the fatty acid ester relative to fat/oil can be increased.

The acrolein production method of the present invention is not limited to the first exemplary embodiment and the second exemplary embodiment mentioned above. In the above-mentioned exemplary embodiments, a part or a whole of the residue remaining after the fourth step is returned back to the first step. However, the fatty acid ester can also be produced not by returning the fatty acid remaining after the fourth step back to the first step, but by reacting it with an alcohol.

The alcohol usable herein is the same as that exemplified in the first step.

Upon the reaction of the fatty acid with the alcohol, it is preferable to use an esterification catalyst. The esterification catalyst includes acid catalysts and basic catalysts. The acid catalysts can be exemplified by inorganic acids such as sulfuric acid, hydrochloric acid, and phosphoric acid; strong acid ion-exchange resins; heteropoly acids such as silicotungstic acid and phosphotungstic acid; and zirconium sulphate. The basic catalysts can be exemplified by hydroxides, oxides, carbonates, or alkoxides of such alkali metals as sodium, potassium, rubidium, and cesium;, hydroxides, oxides, carbonates, or alkoxides of such alkali earth metals as magnesium, calcium, strontium, and barium; strong basic anion-exchange resins, and amines.

Moreover, a fatty acid alkaline salt can also be produced by reacting the fatty acid remaining after the fourth step with an alkali.

The alkali used for producing the fatty acid alkaline salt can be exemplified by a hydroxide or a carbonate of sodium, potassium, magnesium, or calcium.

The produced fatty acid alkaline salt can be used as soap.

### [Acrylic acid production method]

Next is a description of one exemplary embodiment of the acrylic acid production method of the present invention.

The acrylic acid production method of this exemplary embodiment is a method for producing acrylic acid by a) carrying out the above-mentioned acrolein production method and b) reacting the acrolein produced in step a) with molecular oxygen.

In the oxidation reaction, it is preferable to use an oxidation catalyst for accelerating the reaction speed. The oxidation catalyst can be exemplified by solid catalysts including metal oxides, a mixture or oxide complex thereof, or the like. The metal oxide can be made of one or more types of metal(s) selected from the group consisting of iron, molybdenum, titanium, vanadium, tungsten, antimony, tin, and copper.

The oxidation catalyst may be a supported catalyst in which the oxide is held by a carrier. The carrier can be exemplified by silica, alumina, zirconia, a mixture or oxide complex thereof, silicon carbide, or the like.

The form of the catalyst is not specifically limited, and examples thereof can include a powder form, a globular form, a columnar form, a saddle form, and a honeycomb form.

The method for preparing the catalyst can be exemplified by an impregnation method, a precipitation method, and an ion exchange method.

Moreover, the catalyst may also be previously baked in gas according to the purpose. Examples of the gas can include nitrogen, argon, helium, and air.

As for the oxidation reaction, for example, a fixed bed vapor phase reaction or a fluidized bed vapor phase reaction is applied.

Preferred temperature for the oxidation reaction is 150 to 400°C, and more preferred temperature is 200 to 350°C, as it offers high reaction efficiency.

Preferred pressure is 0.01 to 10.0 MPa, and more preferred pressure is 0.05 to 10 MPa.

As to the molecular oxygen for the oxidation reaction, the oxidation source that can be used may be either in a form of oxygen per se or in a form of oxygen-containing air.

Upon the oxidation reaction, an inert gas may also be added. The usable inert gas can be exemplified by nitrogen, carbon dioxide, noble gas (such as helium and argon), and water vapor.

The gas composition in the oxidation reaction needs to be adjusted to be out of the explosive range. Examples of such a composition can include compositions having acrolein accounting for 1 to 15% by volume, oxygen accounting for 0.5 to 25% by volume, water vapor accounting for 0 to 50% by volume, and nitrogen accounting for 20 to 80% by volume.

Acrolein produced by the fourth step is normally used for the oxidation reaction. However, gaseous acrolein produced by the third step can also be used for the reaction either directly or in a mixed form with molecular oxygen and an inert gas such as water vapor. In this case, a single or tandem reactor can be used such as those used for the production of acrylic acid from propylene by two-stage vapor phase oxidation.

It is preferable to add a polymerization inhibitor to the acrylic acid produced by the oxidation reaction, so as to prevent polymerization. Usable polymerization inhibitors are the same as those to be added to acrolein. The addition amount of the polymerization inhibitor, if added, is preferably 1 mass ppm to 1 mass % assuming that the amount of the acrylic acid is 100 mass %.

Moreover, the acrylic acid produced by the oxidation reaction is preferably purified since it will be used as a raw material for various chemicals and polymers. The purification method is preferably distillation similarly to the case of purifying acrolein. The applicable distillation method is also similar to the case of distilling acrolein.

Regarding the condition for the distillation, if the acrylic acid is subjected to multistage distillation, the temperature at the bottom of the column is preferably set at 0 to 120°C, more preferably 5 to 100°C, and yet more preferably 10 to 80°C. If the temperature at the bottom of the column is higher than 120°C, acrylic acid may be polymerized. If the temperature at the bottom of the column is lower than 0°C, the energy amount required for cooling tends to be incremented. The pressure of distillation is determined depending on the relation with the temperature.

The acrylic acid production method described above is capable of, similarly to the acrolein production method, producing acrylic acid from the glycerin mixture with a little consumption of energy.

Hereinunder is a more detailed description of the present invention, with reference to examples. However, the present invention is not to be limited by the examples below.

### (Comparative Example 1)

A glycerin mixture (500 g) containing 60% by mass of glycerin, 5.4% by mass of palmitic acid, 5.1% by mass of oleic acid, and 29.5% by mass of other components was charged in a 1000 ml flask equipped with a rectification column, and was subjected to distillation under reduced pressure, by which a glycerin fraction was produced. The amount of the collected distillate was 291 g. This distillate was analyzed by gas chromatography, which showed that the composition ratio of glycerin was 98% by mass with no presence of palmitic acid or oleic acid. The recovery rate of glycerin in the fraction relative to the charged glycerin was 95%.

The glycerin distillate (204 g) and potassium hydrogen sulfate (KHSO₄) (15 g) serving as a dehydration catalyst were charged in a 500 ml flask equipped with a simple distillation tube. Next, the mixture was heated to 280°C under stirring to effect dehydration for three hours, while performing distillation through the simple distillation tube. The distillate was collected from the distillation tube.

The amount of the thus produced distillate was 166 g. This distillate was analyzed by gas chromatography, and thereby was found to contain 73 g of acrolein. The glycerin conversion ratio was 100%, and the yield of acrolein relative to glycerin was 60%.

Next, the produced distillate was subjected to precision distillation, by which 69 g of acrolein was produced. The yield of acrolein relative to glycerin was 57%.

### (Comparative Example 2)

A glycerin mixture (500 g) containing 60% by mass of glycerin, 2.4% by mass of palmitic acid monoglyceride, 2.1% by mass of oleic acid monoglyceride, and 35.5% by mass of other components was charged in a 1000 ml flask equipped with a rectification column, and was subjected to distillation under reduced pressure, by which a glycerin fraction was produced. The amount of the collected distillate was 291 g. This distillate was analyzed by gas chromatography, which showed that the composition ratio of glycerin was 98% by mass with no presence of palmitic acid monoglyceride or oleic acid monoglyceride. The recovery rate of glycerin in the fraction relative to the charged glycerin was 95%.

The glycerin distillate (204 g) and potassium hydrogen sulfate (KHSO₄) (15 g) serving as a dehydration catalyst were charged in a 500 ml flask equipped with a simple distillation tube. Next, the mixture was heated to 280°C under stirring to effect dehydration for three hours, while performing distillation through the simple distillation tube. The distillate was collected from the distillation tube.

The amount of the thus produced distillate was 166 g. This distillate was analyzed by gas chromatography, and thereby was found to contain 73 g of acrolein. The glycerin conversion ratio was 100%, and the yield of acrolein relative to glycerin was 60%.

Next, the produced distillate was subjected to precision distillation, by which 69 g of acrolein was produced. The yield of acrolein relative to glycerin was 57%.

### (Comparative Example 3)

A glycerin mixture (500 g) containing 60% by mass of glycerin, 4.2% by mass of methyl palmitate, 4.0% by mass of methyl oleate, and 31.8% by mass of other components was charged in a 1000 ml flask equipped with a rectification column, and was subjected to distillation under reduced pressure, by which a glycerin fraction was produced. The amount of the collected distillate was 291 g. This distillate was analyzed by gas chromatography, which showed that the composition ratio of glycerin was 98% by mass with no presence of methyl palmitate or methyl oleate. The recovery rate of glycerin in the fraction relative to the charged glycerin was 95%.

The glycerin distillate (204 g) and potassium hydrogen sulfate (KHSO₄) (15 g) serving as a dehydration catalyst were charged in a 500 ml flask equipped with a simple distillation tube. Next, the mixture was heated to 280°C under stirring to effect dehydration for three hours, while performing distillation through the simple distillation tube. The distillate was collected from the distillation tube.

The amount of the thus produced distillate was 166 g. This distillate was analyzed by gas chromatography, and thereby was found to contain 73 g of acrolein. The glycerin conversion ratio was 100%, and the yield of acrolein relative to glycerin was 60%.

Next, the produced distillate was subjected to precision distillation, by which 69 g of acrolein was produced. The yield of acrolein relative to glycerin was 57%.

### [Example 1]

### Liquid phase reaction of acrolein

A glycerin mixture (333 g) containing 60% by mass of glycerin, 5.4% by mass of palmitic acid, 5.1% by mass of oleic acid, and 29.5% by mass of other components, and potassium hydrogen sulfate (15 g) serving as a dehydration catalyst were charged in a 500 ml flask equipped with a simple distillation tube. Next, the mixture was heated to 280°C under stirring to effect dehydration for three hours, while performing distillation through the simple distillation tube. The distillate was collected from the distillation tube.

The amount of the thus produced distillate was 207 g, in which 79 g of acrolein was contained. Meanwhile, a solid matter was separated from the residue remaining in the flask, by which 126 g of residual liquid was left. This residual liquid contained 13% by mass of palmitic acid and 12% by mass of oleic acid.

From the masses of the components contained in the distillate and the residual liquid, the glycerin conversion ratio was calculated and was found to be 100%. The yield of acrolein relative to glycerin was also calculated and was found to be 65%.

Then, the distillate was subjected to precision distillation, by which 75 g of acrolein was produced. The yield of acrolein relative to glycerin was 62%. That is, though the glycerin mixture was not distillated, acrolein was nonetheless able to be produced at an equivalent yield as compared to the comparative example 1 in which the glycerin mixture was purified by distillation.

In addition, since acrolein was able to be produced even without distilling the glycerin mixture at an equivalent yield as compared to the comparative example 1, it was found to be possible to produce acrolein with less consumption of energy per unit quantity of acrolein.

### [Example 2]

### Vapor phase reaction of acrolein

A potassium hydrogen sulfate aqueous solution was prepared by dissolving 6 g of potassium hydrogen sulfate with water. This potassium hydrogen sulfate aqueous solution was impregnated in 14 g of silica. The resulting product was dried and then baked under nitrogen atmosphere at 300°C for three hours, by which a dehydration catalyst made of potassium hydrogen sulfate/silica was obtained.

This dehydration catalyst (5 ml) was packed in a stainless reaction tube having an inner diameter of 10 mm and a length of 300 mm. Then, in this reaction tube, the glycerin mixture used in the example 1 and a same mass of water as that of the glycerin mixture were added, by which a glycerin mixture containing 20% by mass of glycerin, 1.8% by mass of palmitic acid, 1.7% by mass of oleic acid, and 76.5% by mass of other components was newly produced. This glycerin mixture was fed at 8 g/hour and dehydrated at 300°C. At that time, the outlet of the reaction tube was chilled to thereby condense and collect the reacted gas. The collected liquid was analyzed by gas chromatography, which showed that the glycerin conversion ratio was 100%, and the yield of acrolein was 65%.

Then, the collected liquid was subjected to precision distillation, by which purified acrolein was obtained. The yield of acrolein relative to glycerin was 62%. Also, in this case, though the glycerin mixture was not distillated, acrolein was nonetheless able to be produced at an equivalent yield as compared to the comparative example 1 in which the glycerin mixture was purified by distillation.

### [Example 3]

### Vapor phase reaction of acrylic acid

7.0 g of ammonium paramolybdate, 2.1 g of ammonium metavanadate, 0.89 g of ammonium paratungstate, and 50 ml of water were charged in a flask, and the mixture was dissolved by heating at 90°C under stirring. To the thus produced solution was added a copper nitrate aqueous solution that had been previously prepared by dissolving 1.8 g of copper nitrate with 15 ml of water, by which a catalyst preparation solution was produced.

This catalyst preparation solution was impregnated in 20 g of α-alumina, and next the product was evaporated to dryness. After being dried, the resulting product was baked under air atmosphere at 400°C for three hours, by which an oxidation catalyst made of a molybdenum-vanadium-tungsten-copper oxide held by α-alumina was obtained.

The oxidation catalyst (5 ml) was packed in a stainless reaction tube having an inner diameter of 10 mm and a length of 300 mm. Then, in this reaction tube, using the acrolein produced in the example 1, a mixture gas containing 3% by volume of acrolein, 3% by volume of oxygen, 30% by volume of water vapor, and 64% by volume of nitrogen was introduced at a space velocity of 3000 /hour (STP). Moreover, the reaction tube was heated to 280°C in an electric furnace to effect oxidation reaction. At that time, the outlet of the reaction tube was chilled to thereby condense and collect the reacted gas.

The collected liquid was analyzed by gas chromatography, which showed that the acrolein conversion ratio was 98%, and the yield of acrylic acid relative to acrolein was 90%.

### [Example 4]

The residual liquid (10 g) remaining after the collection of acrolein in the example 1 (13% by mass of palmitic acid and 12% by mass of oleic acid), methylalcohol (50 g) and 95% by mass sulfuric acid (0.1 g) serving as an esterification catalyst were charged in a 500 ml flask equipped with a cooling tube. Then, the mixture was reacted at 65°C under stirring for three hours. The thus produced reaction solution was analyzed by gas chromatography, which showed that the palmitic acid conversion ratio was 95%, and the oleic acid conversion ratio was 95%. Moreover, the yield of methyl palmitate relative to palmitic acid was 91%, and the yield of methyl oleate relative to oleic acid was 90%.

### [Example 5]

The residual liquid (10 g) remaining after the collection of acrolein in the example 1 (13% by mass of palmitic acid and 12% by mass of oleic acid) was charged in a 100 ml flask. Then, the liquid was neutralized by adding 10% by mass sodium hydroxide aqueous solution under stirring, by which a soap solution was produced. The soap solution was analyzed by gas chromatography, which showed that there was no presence of palmitic acid or oleic acid, and the reaction was found to be completed.

### [Example 6]

### Liquid phase reaction of acrolein

A glycerin mixture (333 g) containing 60% by mass of glycerin, 2.4% by mass of palmitic acid monoglyceride, 2.1% by mass of oleic acid monoglyceride, and 35.5% by mass of other components, and potassium hydrogen sulfate (15 g) serving as a dehydration catalyst were charged in a 500 ml flask equipped with a simple distillation tube. Next, the mixture was heated to 280°C under stirring to effect dehydration for three hours, while performing distillation through the simple distillation tube. The distillate was collected from the distillation tube.

The amount of the thus produced distillate was 207 g, in which 79 g of acrolein was contained. Meanwhile, a solid matter was separated from the residue remaining in the flask, by which 126 g of residual liquid was left. This residual liquid contained 5.7% by mass of palmitic acid monoglyceride and 5.0% by mass of oleic acid monoglyceride.

From the masses of the components contained in the distillate and the residual liquid, the glycerin conversion ratio was calculated and was found to be 100%. The yield of acrolein relative to glycerin was also calculated and was found to be 65%.

Then, the distillate was subjected to precision distillation, by which 75 g of acrolein was produced. The yield of acrolein relative to glycerin was 62%. That is, though the glycerin mixture was not distillated, acrolein was nonetheless able to be produced at an equivalent yield as compared to the comparative example 2 in which the glycerin mixture was purified by distillation.

In addition, since acrolein was able to be produced even without distilling the glycerin mixture at an equivalent yield as compared to the comparative example 2, it was found to be possible to produce acrolein with less consumption of energy per unit quantity of acrolein.

### [Example 7]

### Vapor phase reaction of acrolein

A potassium hydrogen sulfate aqueous solution was prepared by dissolving 6 g of potassium hydrogen sulfate with water. This potassium hydrogen sulfate aqueous solution was impregnated in 14 g of silica. The resulting product was dried and then baked under nitrogen atmosphere at 300°C for three hours, by which a dehydration catalyst made of potassium hydrogen sulfate/silica was obtained.

This dehydration catalyst (5 ml) was packed in a stainless reaction tube having an inner diameter of 10 mm and a length of 300 mm. Then, in this reaction tube, the glycerin mixture used in the example 6 and a same mass of water as that of the glycerin mixture were added, by which a glycerin mixture containing 20% by mass of glycerin, 0.8% by mass of palmitic acid monoglyceride, 0.7% by mass of oleic acid, and 78.5% by mass of other components was newly produced. This glycerin mixture was fed at 8 g/hour and dehydrated at 300°C. At that time, the outlet of the reaction tube was chilled to thereby condense and collect the reacted gas. The collected liquid was analyzed by gas chromatography, which showed that the glycerin conversion ratio was 100%, and the yield of acrolein was 65%.

Then, the collected liquid was subjected to precision distillation, by which purified acrolein was obtained. The yield of acrolein relative to glycerin was 62%. Also, in this case, though the glycerin mixture was not distillated, acrolein was nonetheless able to be produced at an equivalent yield as compared to the comparative example 1 in which the glycerin mixture was purified by distillation.

### [Example 8]

### Vapor phase reaction of acrylic acid

7.0 g of ammonium paramolybdate, 2.1 g of ammonium metavanadate, 0.89 g of ammonium paratungstate, and 50 ml of water were charged in a flask, and the mixture was dissolved by heating at 90°C under stirring. To the thus produced solution was added a copper nitrate aqueous solution that had been previously prepared by dissolving 1.8 g of copper nitrate with 15 ml of water, by which a catalyst preparation solution was produced.

This catalyst preparation solution was impregnated in 20 g of α-alumina, and next the product was evaporated to dryness. After being dried, the resulting product was baked under air atmosphere at 400°C for three hours, by which an oxidation catalyst made of a molybdenum-vanadium-tungsten-copper oxide held by α-alumina was obtained.

The oxidation catalyst (5 ml) was packed in a stainless reaction tube having an inner diameter of 10 mm and a length of 300 mm. Then, in this reaction tube, using the acrolein produced in the example 6, a mixture gas containing 3% by volume of acrolein, 3% by volume of oxygen, 30% by volume of water vapor, and 64% by volume of nitrogen was introduced at a space velocity of 3000 /hour (STP). Moreover, the reaction tube was heated to 280°C in an electric furnace to effect oxidation reaction. At that time, the outlet of the reaction tube was chilled to thereby condense and collect the reacted gas.

The collected liquid was analyzed by gas chromatography, which showed that the acrolein conversion ratio was 98%, and the yield of acrylic acid relative to acrolein was 90%.

### [Example 9]

The residual liquid (10 g) remaining after the collection of acrolein in the example 6 (5.7% by mass of palmitic acid monoglyceride and 5.0% by mass of oleic acid monoglyceride), methylalcohol (50 g) and 95% by mass sulfuric acid (0.1 g) serving as an esterification catalyst were charged in a 500 ml flask equipped with a cooling tube. Then, the mixture was reacted at 65°C under stirring for three hours. The thus produced reaction solution was analyzed by gas chromatography, which showed that the conversion ratio of palmitic acid monoglyceride was 94% and the conversion ratio of oleic acid monoglyceride was 94%. Moreover, the yield of methyl palmitate relative to palmitic acid monoglyceride was 94%, and the yield of methyl oleate relative to oleic acid monoglyceride was 93%.

### [Example 10]

The residual liquid (10 g) remaining after the collection of acrolein in the example 6 (5.7% by mass of palmitic acid monoglycerides and 5.0% by mass of oleic acid monoglyceride) was charged in a 100 ml flask. Then, the liquid was neutralized by adding 10% by mass sodium hydroxide aqueous solution under stirring, by which a soap solution was produced. The soap solution was analyzed by gas chromatography, which showed that there was no presence of palmitic acid monoglyceride or oleic acid monoglyceride, and the reaction was found to be completed.

### [Example 11]

A glycerin mixture (333 g) containing 50 g of the residual liquid remaining after the collection of acrolein in the example 6 (5.7% by mass of palmitic acid monoglycerides and 5.0% by mass of oleic acid monoglyceride), 60% by mass of glycerin, 2.4% by mass of palmitic acid monoglyceride, 2.1% by mass of oleic acid monoglyceride, and 35.5% by mass of other components, and potassium hydrogen sulfate (15 g) serving as a dehydration catalyst were charged in a 500 ml flask equipped with a simple distillation tube. Next, the mixture was heated to 280°C under stirring to effect dehydration for three hours, while performing distillation through the simple distillation tube. The distillate was collected from the distillation tube.

The amount of the thus produced distillate was 216 g, in which 81 g of acrolein was contained. Meanwhile, a solid matter was separated from the residue remaining in the flask, by which 167 g of residual liquid was left. This residual liquid contained 8.6% by mass of palmitic acid monoglyceride and 7.5% by mass of oleic acid monoglyceride.

From the masses of the components contained in the distillate and the residual liquid, the glycerin conversion ratio was calculated and was found to be 100%. The yield of acrolein relative to glycerin was also calculated and was found to be 67%.

Then, the distillate was subjected to precision distillation, by which 77 g of acrolein was produced. The yield of acrolein relative to glycerin was 63%.

### [Example 12]

### Liquid phase reaction of acrolein

A glycerin mixture (333 g) containing 60% by mass of glycerin, 4.2% by mass of methyl palmitate, 4.0% by mass of methyl oleate, and 31.8% by mass of other components, and potassium hydrogen sulfate (15 g) as a dehydration catalyst were charged in a 500 ml flask equipped with a simple distillation tube. Next, the mixture was heated to 280°C under stirring to effect dehydration for three hours, while performing distillation through the simple distillation tube. The distillate was collected from the distillation tube.

The amount of the thus produced distillate was 207 g, in which 79 g of acrolein was contained. Meanwhile, a solid matter was separated from the residue remaining in the flask, by which 126 g of residual liquid was left. This residual liquid contained 10% by mass of methyl palmitate and 9.5% by mass of methyl oleate.

From the masses of the components contained in the distillate and the residual liquid, the glycerin conversion ratio was calculated and was found to be 100%. The yield of acrolein relative to glycerin was also calculated and was found to be 65%.

Then, the distillate was subjected to precision distillation, by which 75 g of acrolein was produced. The yield of acrolein relative to glycerin was 62%. That is, though the glycerin mixture was not distillated, acrolein was nonetheless able to be produced at an equivalent yield as compared to the comparative example 3 in which the glycerin mixture was purified by distillation.

In addition, since acrolein was able to be produced even without distilling the glycerin mixture at an equivalent yield as compared to the comparative example 3, it was found to be possible to produce acrolein with less consumption of energy per unit quantity of acrolein.

### [Example 13]

### Vapor phase reaction of acrolein

A potassium hydrogen sulfate aqueous solution was prepared by dissolving 6 g of potassium hydrogen sulfate with water. This potassium hydrogen sulfate aqueous solution was impregnated in 14 g of silica. The resulting product was dried and then baked under nitrogen atmosphere at 300°C for three hours, by which a dehydration catalyst made of potassium hydrogen sulfate/silica was obtained.

This dehydration catalyst (5 ml) was packed in a stainless reaction tube having an inner diameter of 10 mm and a length of 300 mm. Then, in this reaction tube, the glycerin mixture used in the example 12 and a same mass of water as that of the glycerin mixture were added, by which a glycerin mixture containing 20% by mass of glycerin, 1.4% by mass of methyl palmitate, 1.3% by mass of methyl oleate, and 77.3% by mass of other components was newly produced. This glycerin mixture was fed at 8 g/hour and dehydrated at 300°C. At that time, the outlet of the reaction tube was chilled to thereby condense and collect the reacted gas. The collected liquid was analyzed by gas chromatography, which showed that the glycerin conversion ratio was 100%, and the yield of acrolein was 65%.

Then, the collected liquid was subjected to precision distillation, by which purified acrolein was obtained. The yield of acrolein relative to glycerin was 62%. Also, in this case, though the glycerin mixture was not distillated, acrolein was nonetheless able to be produced at an equivalent yield as compared to the comparative example 3 in which the glycerin mixture was purified by distillation.

### [Example 14]

### Vapor phase reaction of acrylic acid

7.0 g of ammonium paramolybdate, 2.1 g of ammonium metavanadate, 0.89 g of ammonium paratungstate, and 50 ml of water were charged in a flask, and the mixture was dissolved by heating at 90°C under stirring. To the thus produced solution was added a copper nitrate aqueous solution that had been previously prepared by dissolving 1.8 g of copper nitrate with 15 ml of water, by which a catalyst preparation solution was produced.

This catalyst preparation solution was impregnated in 20 g of α-alumina, and next the product was evaporated to dryness. After being dried, the resulting product was baked under air atmosphere at 400°C for three hours, by which an oxidation catalyst made of a molybdenum-vanadium-tungsten-copper oxide held by α-alumina was obtained.

The oxidation catalyst (5 ml) was packed in a stainless reaction tube having an inner diameter of 10 mm and a length of 300 mm. Then, in this reaction tube, using the acrolein produced in the example 12, a mixture gas containing 3% by volume of acrolein, 3% by volume of oxygen, 30% by volume of water vapor, and 64% by volume of nitrogen was introduced at a space velocity of 3000/hour (STP). Moreover, the reaction tube was heated to 280°C in an electric furnace to effect oxidation reaction. At that time, the outlet of the reaction tube was chilled to thereby condense and collect the reacted gas.

The collected liquid was analyzed by gas chromatography, which showed that the acrolein conversion ratio was 98%, and the yield of acrylic acid relative to acrolein was 90%.

## Claims

1. An acrolein production method comprising: performing, either concurrently or sequentially,
(1) a step of dehydrating a glycerin mixture comprising the following (i) and (ii):
(i) 5 to 95 % by mass of glycerin; and
(ii) at least one selected from the group consisting of: a glyceride; either one or both of a fatty acid and a fatty acid salt; and a fatty acid ester,
to produce an acrolein mixture comprising the following (iii) and (iv):
(iii) acrolein; and
(iv) at least one selected from the group consisting of: a glyceride; a fatty acid ester; and either one or both of a fatty acid and a fatty acid salt; and
(2) a step of collecting acrolein from the acrolein mixture;
wherein the acrolein production method further comprises, prior to said step (1) of producing the acrolein mixture:
(a) a step of transesterifying a fat/oil and an alcohol, to produce a fatty acid ester mixture comprising: a fatty acid ester; and at least any one of glycerin, a glyceride, and either one or both of a fatty acid and a fatty acid salt; and
(b) a step of removing the fatty acid ester from the fatty acid ester mixture, to thereby produce the glycerin mixture, in which the mass ratio of fatty acid ester to glycerin is 0.001-1:1; and
after said step (2) of collecting acrolein from the acrolein mixture:
treating a part or a whole of the remaining residue with an acid and returning the treated residue to the step (a) of producing the fatty acid ester mixture;
or
wherein the acrolein production method further comprises, prior to said step (1) of producing the acrolein mixture:
(c) a step of saponifying a fat/oil and an alkali, to produce a fatty acid alkaline salt mixture comprising a fatty acid alkaline salt; glycerin, and either one or both of a fatty acid and a fatty acid salt; and
(d) a step of removing the fatty acid alkaline salt from the fatty acid alkaline salt mixture, to produce the glycerin mixture, in which the ratio of the fatty acid alkaline salt to glycerin is 0.001-1:1; and
after said step (2) of collecting acrolein from the acrolein mixture:
returning a part or a whole of the remaining residue to the step (c) of producing the fatty acid alkaline salt mixture.

2. An acrolein production method according to claim 1, wherein the step (2) of collecting acrolein from the acrolein mixture is carried out by distillation of the acrolein mixture.

3. An acrolein production method according to claim 1 or 2, wherein said fatty acid comprises one or more types of fatty acid(s) selected from the group consisting of C4 to C24 fatty acids; said fatty acid salt comprises salt(s) of one or more types of fatty acid(s) selected from the group consisting of C4 to C24 fatty acids, and one or more types of compound(s) selected from the group consisting of an alkali metal compound, an alkali earth metal compound, and an amine compound; and the fatty acid constituting said glyceride comprises one or more types of fatty acid(s) selected from the group consisting of C4 to C24 fatty acids.

4. An acrylic acid production method, comprising a) the acrolein production method according to any one of claims 1 to 3 and b) reacting the acrolein produced in step a) with molecular oxygen.

## Patentansprüche

1. Verfahren zur Herstellung von Acrolein, welches umfasst: das entweder gleichzeitige oder aufeinanderfolgende Durchführen
(1) einer Stufe, in der ein (i) und (ii) umfassendes Glyceringemisch dehydriert wird:
(i) 5 bis 95 Massen-% Glycerin; und
(ii) mindestens ein aus der Gruppe ausgewähltes Mitglied, die aus folgenden Elementen besteht: einem Glycerid; einer Fettsäure und/oder einem Fettsäuresalz; und einem Fettsäureester,
wobei ein (iii) und (iv) umfassendes Acroleingemisch hergestellt wird:
(iii) Acrolein; und
(iv) mindestens ein aus der Gruppe ausgewähltes Mitglied, die aus folgenden Elementen besteht: einem Glycerid; einem Fettsäureester; und einer Fettsäure und/oder einem Fettsäuresalz; und
(2) eine Stufe, in der Acrolein aus dem Acroleingemisch gewonnen wird;
wobei das Verfahren zur Herstellung von Acrolein außerdem vor der Stufe (1) der Herstellung des Acroleingemisches umfasst:
(a) eine Stufe, in der ein Fett/Öl und ein Alkohol transesterifiziert werden, wobei ein Fettsäureestergemisch hergestellt wird, welches umfasst: einen Fettsäureester; und mindestens ein Mitglied der Gruppe, die aus Glycerin, einem Glycerin und Fettsäure und/oder Fettsäuresalz besteht; und
(b) eine Stufe, in der der Fettsäureester aus dem Fettsäureestergemisch entfernt wird, um das Glyceringemisch zu bilden, wobei das Massenverhältnis von Fettsäureester zu Glycerin 0,001 - 1:1 ist; und
nach der Stufe (2), in der Acrolein aus dem Acroleingemisch gewonnen wird:
die Behandlung eines Teils oder des gesamten verbleibenden Rests mit einer Säure und das Rückführen des behandelten Rests in die Stufe (a) zur Herstellung des Fettsäureestergemisches;
wobei das Verfahren zur Herstellung von Acrolein außerdem vor der Stufe (1) zur Herstellung des Acroleingemisches umfasst:
(c) eine Stufe, in der ein Fett/Öl und ein Alkali verseift werden, wobei ein Fettsäurealkalisalzgemisch erhalten wird, das ein Fettsäurealkalisalz; Glycerin und Fettsäure und/oder Fettsäuresalz umfasst; und
(d) eine Stufe, in der das Fettsäurealkalisalz aus dem Fettsäurealkalisalzgemisch unter Bildung des Glyceringemisches entfernt wird, wobei das Verhältnis des Fettsäurealkalisalzes zu Glycerin 0,001 - 1:1 ist; und
nach der Stufe (2) des Gewinnens von Acrolein aus dem Acroleingemisch:
das Zurückführen eines Teils oder des gesamten verbleibenden Rests in die Stufe (c) zur Herstellung des Fettsäurealkalisalzgemisches.

2. Verfahren zur Herstellung von Acrolein nach Anspruch 1, wobei die Stufe (2) des Gewinnens von Acrolein aus dem Acroleingemisch durch Destillation des Acroleingemisches durchgeführt wird.

3. Verfahren zur Herstellung von Acrolein nach Anspruch 1 oder 2, wobei die Fettsäure eine oder mehrere Arten von Fettsäure(n) enthält, ausgewählt aus der Gruppe, die aus C4- bis C24-Fettsäuren besteht; das Fettsäuresalz ein oder mehrere Salze einer oder mehrere Arten von Fettsäure(n), ausgewählt aus der Gruppe, die aus C4- bis C24-Fettsäuren besteht, und eine oder mehrere Arten von Verbindung(en) enthält, ausgewählt aus der Gruppe, die aus einer Alkalimetallverbindung, einer Erdalkalimetallverbindung und einer Aminverbindung besteht; und die Fettsäure des Glycerids ein oder mehrere Arten von Fettsäure(n) enthält, ausgewählt aus der Gruppe, die aus C4- bis C24-Fettsäuren besteht.

4. Verfahren zur Herstellung von Acrylsäure, welches a) das Verfahren zur Herstellung von Acrolein nach einem der Ansprüche 1 bis 3 und b) das Umsetzen des in Stufe a) hergestellten Acroleins mit molekularem Sauerstoff umfasst.

## Revendications

1. Procédé de production d'acroléine comprenant : la réalisation soit concurremment soit successivement,
(1) d'une étape de déshydratation d'un mélange de glycérine comprenant les (i) et (ii) suivants :
(i) de 5 à 95 % en masse de glycérine ; et
(ii) d'au moins un choisi dans le groupe constitué de : un glycéride ; soit l'un soit les deux d'un acide gras et d'un sel d'acide gras ; et un ester d'acide gras,
pour produire un mélange d'acroléine comprenant les (iii) et (iv) suivants :
(iii) l'acroléine ; et
(iv) au moins un choisi dans le groupe constitué de : un glycéride ; un ester d'acide gras ; et soit l'un soit les deux d'un acide gras et d'un sel d'acide gras ; et
(2) d'une étape de recueil d'acroléine à partir du mélange d'acroléine ;
dans lequel le procédé de production d'acroléine comprend de plus, avant ladite étape (1) de production du mélange d'acroléine :
(a) une étape de transestérification d'une graisse/huile et d'un alcool, pour produire un mélange d'ester d'acide gras comprenant : un ester d'acide gras ; et au moins l'un parmi la glycérine, un glycéride, et soit l'un soit les deux d'un acide gras et d'un sel d'acide gras ; et
(b) une étape d'élimination de l'ester d'acide gras du mélange d'ester d'acide gras, pour produire par-là le mélange de glycérine, dans lequel le rapport massique d'ester d'acide gras à glycérine est de 0,001-1:1 ; et
après ladite étape (2) de recueil d'acroléine du mélange d'acroléine :
de traitement d'une partie ou de la totalité du résidu restant avec un acide et de renvoi du résidu traité dans l'étape (a) de production du mélange d'ester d'acide gras ;
dans lequel le procédé de production d'acroléine comprend de plus, avant ladite étape (1) de production du mélange d'acroléine :
(c) une étape de saponification d'une graisse/huile et d'un alcali, pour produire un mélange de sel alcalin d'acide gras comprenant un sel alcalin d'acide gras ; de la glycérine, et soit l'un soit les deux d'un acide gras et d'un sel d'acide gras ; et
(d) une étape d'élimination du sel alcalin d'acide gras du mélange de sel alcalin d'acide gras, pour produire le mélange de glycérine, dans laquelle le rapport du sel alcalin d'acide gras à la glycérine est de 0,001-1:1 ; et
après ladite étape (2) de recueil d'acroléine du mélange d'acroléine :
de renvoi d'une partie ou de la totalité du résidu restant dans l'étape (c) de production du mélange de sel alcalin d'acide gras.

2. Procédé de production d'acroléine selon la revendication 1, dans lequel l'étape (2) de recueil d'acroléine du mélange d'acroléine est réalisée par distillation du mélange d'acroléine.

3. Procédé de production d'acroléine selon la revendication 1 ou 2, dans lequel ledit acide gras comprend un ou plusieurs types d'acide(s) gras choisi(s) dans le groupe constitué d'acides gras en C4 à C24 ; ledit sel d'acide gras comprend un(des) sel(s) d'un ou plusieurs types d'acide(s) gras choisi(s) dans le groupe constitué d'acides gras en C4 à C24, et un ou plusieurs types de composé(s) choisi(s) dans le groupe constitué d'un composé de métal alcalin, d'un composé de métal alcalino-terreux, et d'un composé d'amine ; et l'acide gras constituant ledit glycéride comprend un ou plusieurs types d'acide(s) gras choisi(s) dans le groupe constitué d'acides gras en C4 à C24.

4. Procédé de production d'acide acrylique, comprenant a) le procédé de production d'acroléine selon l'une quelconque des revendications 1 à 3 et b) la réaction de l'acroléine produite dans l'étape a) avec de l'oxygène moléculaire.
